# EUROPEAN PATENT SPECIFICATION

(11) **EP 2 277 577 B1**
(45) Date of publication and mention of the grant of the patent: **14.03.2018**
(21) Application number: 10010487.6
(22) Date of filing: 06.10.2005
(51) Int. Cl.: A61M 11/00, A61M 15/00, A61M 11/02

(54) **Dispensing device for dispensing powder**
Abgabevorrichtung zur Abgabe von Pulver
Dispositif de distribution de poudre

(30) Priority: 06.10.2004 GB 0422106; 17.11.2004 GB 0425289; 15.12.2004 GB 0427552; 04.05.2005 GB 0509108
(43) Date of publication of application: 26.01.2011
(62) Divisional of application: 05797355.4
(73) Proprietor: BOEHRINGER INGELHEIM INTERNATIONAL GMBH, 55216 Ingelheim am Rhein (DE)
(72) Inventor: Dunne, Stephen Terence, 55216 Ingelheim am Rhein (DE); Rohschneider, Marc, 55216 Ingelheim am Rhein (DE)
(74) Representative: Von Rohr Patentanwälte Partnerschaft mbB

(56) References cited:
- WO-A-02/053215
- WO-A2-2004/093848
- GB-A- 2 340 758
- US-A- 5 239 991

## Description

The present invention relates to a dispensing device for dispensing powder according to the preamble of claim 1.

Powder drugs delivered through dispensing devices, in particular inhalers, are intended to optimally target specific sites in the pulmonary system. These sites include the nasal passages, the throat, and various locations within the lungs, such as the bronchi, bronchioles and alveolar regions. The ability to deliver drugs to a target area depends inter alia on the aerodynamic sizes of the particles. As currently believed to be understood, particles having an aerodynamic diameter of less than 2 µm are considered to be potentially optimal for deposition in the alveolar region of the lung. Particles that have an aerodynamic diameter of between 2 and approximately 5 µm may be more suitable for delivery to the bronchiole or bronchi regions. Particles with an aerodynamic size range greater than 6 µm, and more preferably 10 µm. are typically suitable for delivery to the laryngeal region, throat or nasal passages.
In most cases, it is desired to achieve a high inhalable fraction and a high delivery efficiency, i.e. the fraction that reaches the desired region, in particular in the lung. This depends on various factors, in particular on the characteristics of the generated spray plume, such as propagation velocity of the plume, particle size and its distribution, fraction of small particles, fraction of gas and the like. The desired spray plume characteristics include preferably a small particle size, a high fraction of drug particles with a diameter of 6 µm or less, a low propagation velocity, a long duration of spray generation and/or possible inhalation, and/or a low amount of gas volume required for dispensing a certain amount of powder.
In particular, the present invention is concerned with dry powder inhalers for the delivery of drugs to the lungs. Many dry powder inhalers are on the market or have been proposed. There are two main types, namely the passive ones and the active ones. In passive inhalers all the energy required for de-agglomerating the powder and transferring the powder to the lungs is provided by the breathing of a user or patient. In active inhalers there is an additional source of energy to help to de-agglomerate the powder.

Most powder inhalers are of the passive type where the powder is inhaled by the patient without the aid of an additional energy source. The problem with passive inhalers is that the inhalable fraction, or the proportion of powder that actually enters the lungs, is largely dependant on the breathing of the patient. The de-agglomeration of the powder and hence the inhalable fraction is a function of the flow rate of inhaled air through the device and, therefore, varies greatly from patient to patient.

Dry powder inhalers are subdivided into single dose devices and multi-douse inhalers. Multi-dose inhalers are further subdivided into pre-metered types where the doses are stored individually and into metering inhalers where the powder dose is metered in the device.

Multi dose pre-metered inhalers have the advantage that the single doses are metered under strict factory conditions and the powder can quite easily be isolated from the atmosphere. In many applications the active duct powder is mixed with a carrier such as lactose which tends to absorb humidity from the atmosphere which makes it stick together and diftucult to de-agglomerate.

The present invention relates to an active, gas powered, pre-metered multi- or single-dose dispensing device for dispensing powder containing or consisting of a drug, such as a dry powder inhaler.

WO 02/053215 A2 discloses a dry powder inhaler having a housing, at least one single dose storage chamber able to contain a single unit dose, a discharge path adjacent the storage chamber, and a flexible and bendable seal plate closing the discharge path. When an air flow under pressure is supplied to the top of the dose, the top seal is broken and the dose is driven against the seal plate which deflects away, allowing the powder of the dose to be driven into and along the discharge path. The air flow causes the seal pilate (in the discharge path) to vibrate, which acts to break up the dose into particles of preferred size, as the dose is carried along with the air flow and delivered to a patient. This dry powder inhaler does not result in optimized de-agglomeration of the powder and/or desired spray plume characteristics. The storage chamber is not designed and not used as a mixing chamber in which swirls or eddies are generated to mix the powder with the air.

WO 92/12799 A1 discloses a pre-metered dispensing device for transforming a flow of fluid into a spray of fine particle size, wherein an annular flow through a duct is caused with a velocity gradient within that flow sufficient to cause sheer forces between components of the flow to break the flow up into a spray. The cross sectional shape of the duct is preferably circular, but other cross sectional shapes, for example an irregular cross section or a polygonal cross section can be used. However, the known device and method are not optimal for de-agglomerating the powder and for generating a slow spray plume with the desired characteristics.

WO 2004/041326 A2 discloses a tubular nozzle for use in systems for delivering medicaments, in particular for metering devices for dispensing liquid aerosols, i.e. so-called metered dose inhalers (MDIs). The tubular nozzle is curvilinear throughout a defined length and has a curved portion with a radius of curvature of at least 2.5 times the inner diameter of the tubular nozzle. The cross section of the tubular nozzle may be selected from a wide range of choices such as circular, oval, square, rectangular, polygonal, and the like.

WO 2004/093848 A2 relates to devices for enhancing dosing efficiency. An active powder inhaler comprises a vortex chamber or nozzle having an exit port and an inlet port for generating an aerosol. Pressurized air is feeded into a powder storage chamber. A mixture of powder and gas is directed by a drug feed tube to the vortex chamber or nozzle. The nozzle is located within a mouthpiece. Further, a storage chamber is disclosed having a tangential inlet and outlet, wherein inlet and outlet are arranged on opposite ends of the chamber. In another embodiments a medicament pack comprises a storage chamber, a drug feed tube, an aerosoling nozzle or vortex chamber arranged in one blister modul.

GB 2 340 758 A discloses a drug dispensing system. The dispensing device comprises multiple dosage chambers. In one embodiment two chambers are connected by a tangentially arranged duct, wherein the first chamber comprises an axially inlet and the second chamber comprises an axially outlet.

US 5 239 991 A relates to a powder inhalation device with a peel-off cover. The device comprises a reservoir for the powder, air inlet means and medicament outlet means. The raised reservoir acts as a mouthpiece. During inhalation an air flow is provided through the axially arranged inlet holes into two passageways, which join the reservoir tangentially. The mixture of gas and powder is dispensed through axially arranged outlet holes.

Object of the present invention is to provide an improved storage device and dispensing device, in particular wherein better de-agglomeration of the powder and/or the desired spray plume characteristics can be realized.

The above object is achieved by a dispensing device according to claim 1. Preferred embodiments are subject of the subclaims.

The dispensing device or the storage device is adapted for dispensing subsequently multiples doses of powder. Each dose uses a different duct. In this way built up of powder in duct corners or blocking does not affect performance.

One aspect is to provide a duct with a flat cross section. The powder is forced through the duct by pressurized gas to de-agglomerate the powder and to generate a spray including fine powder particles. The ratio of the largest side to the smallest side of the flat cross section of the duct is at least 2.0. Surprisingly a much better de-agglomeration and finer particles can be achieved, in particular with a lower amount of gas for a given volume or mass of powder, than by a circular or quasi circular duct. This effect may be explained in that the flat cross section provides a larger perimeter for a given cross sectional area than a non-flat cross section. This larger perimeter results in a larger duct surface that is in contact with the gas and powder so that better de-agglomeration can be achieved due to higher sheer forces without changing the cross sectional area (hydraulic diameter), i.e. without changing the flow resistance or mass flow significantly. Preferably, the ratio of the largest side to the smallest side of the flat cross section is between 3 to 50, most preferably about 5 to 30. Thus, a high output of powder with good de-agglomeration as a spray with small powder particles size can be achieved by a comparatively low gas pressure, low gas volume, and low gas flow rate. The dispensing device produces a plume of de-agglomerated dry powder with a high inhalable fraction and with the desired spray plume characteristics.
It was found that with fine powders of mean particles of fewer than 5 µm a substantially rectangular duct of typically 75 µm by 1500 µm works well. With powders of mean particle size above 30 µm a duct of typically 200 µm by 1500 µm works well. The non circular duct should preferably have a hydraulic diameter of between 20 to 1000 µm depending on the particle size of the powder. It can be made of any material that is drug compatible including plastics or metals. More than one non circular duct may be used in parallel.

The non circular duct preferably has a length of at least 5 or 10, preferably between 10 and 60, hydraulic diameters (the hydraulic diameter is defined as the ratio of 4 cross sectional areas over the duct perimeter). For any given pressure the longer the non circular duct the slower is the powder delivery to the patent: However if the duct is too long the velocity in the storing / mixing chamber may be reduced to an extent that the mixing chamber is not emptied. In particular, it is possible to force the powder through the duct by a gas pressure of less than 300 kPa to de-agglomerate the powder and to generate the spray with fine particle size. So optimal spray plume characteristics, in particular a low propagation velocity, can be achieved.

It is advantageous to minimize the exit velocity of the gas and powder in order to minimize powder impaction in the mouth and upper respiratory tract. However the higher the exit velocity the better is the powder break up or de-agglomeration. One solution to this is to slow the exit velocity of the gas and powder mixture at the duct exit by using two or more impinging ducts or powder jets preferably impinging at an angle of between 30 and 180, preferably 90 and 150, degrees. In particular, multiple - at least two - powder spray jets are impinged, i.e. hit each other, to slow down the propagation velocity of the spray and/or to de-agglomerate the powder. This supports the desired spray plume characteristics as mentioned above. Alternatively a diffuser with an increasing cross section may be used to decelerate the gas and powder flow at the exit of the duct. Any gas may be used. For instance liquefied gases such as HFA134a and HFA227 may be used. In such a device the gas is stored in a pressurized canister containing a metering valve with connecting means to the powder reservoir(s). Alternatively a piston cylinder arrangement, a bellows or any other gas pump may be used to pressurize e.g. atmospheric air. In such a device the user or patient needs to cock or prime the device prior to use. Further, compressed gas may be used. For single doses devices a pre-pressurized canister of compressed air may be used.

The volume of gas needed to completely empty a storage chamber (reservoir) and/or mixing chamber depends on the powder volume or mass. For powder masses of 0.1 to 50 mg, gas masses of between 0.2 and 300 mg are required. For instance 5 mg of powder with a mean particle size of 4 µm requires between 10 and 20 cm³ of compressed air at between 100 kPa and 200 kPa with a mass of approximately 20 to 60 mg of air. For coarser powders less gas volume is needed at lower pressure typically under 100 kPa gauge as less energy for de-agglomeration is required.

The volume of the storage chamber (reservoir) and optional mixing chamber needed to expel all the powder depends on the powder volume or mass. It should preferably have a volume of between 0.002 and 0.2 cm³ depending on powder dose. The larger the powder dose the larger the reservoir/mixing chamber should be. For instance with a powder dose of 5 mg a volume of between 0.015 and 0.03 cm³ is needed for thorough mixing. Preferably, the ratio of the chamber volume (volume of the storage chamber and of the optional mixing chamber) to the powder volume should be between 1.2 and 4.

The reservoir should preferably be of cylindrical shape with no sharp edges as these can attract powder deposits. The gas inlet or inlets should preferably be positioned so that the gas sweeps all the chamber surfaces to prevent powder accumulating on said surfaces. Preferably the inlet(s) should be placed near the chamber end furthest from the outlet, i.e. the non circular duct. The relative positions of the entry(s) and outlet in the reservoir and mixing chamber may be arranged in such a way that the gas powder mixture forms turbulent eddies within the chamber to maximize de-agglomeration or that a smooth non turbulent flow is achieved in the chamber(s).

Preferably surface areas after the non circular duct are minimized to minimize powder adherence or loss on said surfaces. The invention has the advantage that little or no powder is retained in the device after inhalation and hence the metered and delivered masses are almost the same.

The preferably non circular duct is located at a mouthpiece entrance with no flow restrictions after the non circular duct.

The storage chamber for a dose of powder may be closed by a bursting element or another pressure sensitive element designed such that the pressurized gas can burst the bursting element or open the pressure sensitive element for discharging powder from the storage chamber. In particular, the mixture of gas and powder only passes through the preferably non-circular duct, a nozzle or the like after the gas pressure has reached a peak value, wherein the mixture is discharged by a lower gas pressure. This may be achieved by using the pressure sensitive element, such as a valve, the bursting element, a diaphragm or the like.
Preferably, the bursting element is made of a thin plastic film, coated aluminum film or any other suitable material. The pressure sensitive element, e.g. the bursting element, may be placed before the storage chamber or after it, in particular between the storage chamber for the powder and an adjacent mixing chamber.
Preferably, a separate pressure sensitive element or bursting element is used for each dose of powder, i.e. each storage chamber.
According to another aspect of the present invention, the powder is forced through the duct by a comparatively low gas pressure of less than 300 kPa to de-agglomerate the powder and/or to generate the spray. Experiments have shown that such low pressures are sufficient for achieving good de-agglomeration and optimal for achieving a slow spray.
According to another aspect, the gas flow is restricted or controlled at the inlet side, and not at the outlet side during dispensing of the powder. This is in particular possible with the non-circular duct. Preferably, the gas inlet has a respectively small cross section that restricts or controls the gas flow during dispensing.

According to a further aspect, the dispensing device may contain at least two or three separate storage chambers for separate or different drugs/powders, which can be dispensed subsequently or simultaneously and, in the latter case, preferably mixed only during the dispensing.
According to a further aspect of the present invention, the separate drugs or powders can be mixed by impinging at least two powder jets.
Further aspects, advantages and features of the present invention will be an apparent from the claims and following detailed description of preferred embodiments. In the drawings show:
- Fig. 1: a schematic sectional view of a dispensing device;
- Fig. 2: a schematic sectional view of the dispensing device according to Fig. 1 during dispensing;
- Fig. 3: a schematic longitudinal sectional view of a duct with a nozzle;
- Fig. 4: a partial sectional view of a storage chamber and a mixing chamber separated by a bursting element;
- Fig. 5: a schematic sectional view of a storage device with a duct;
- Fig. 6a - 6c: cross sectional views of ducts with different cross sections;
- Fig. 7: a schematic sectional view of a storage device with two ducts;
- Fig. 8: a schematic sectional view of a storage device and an associated cover with two ducts;
- Fig. 9: a schematic sectional view of a storage device with multiple ducts;
- Fig. 10: a schematic cross sectional view of a storage device without bursting element;
- Fig. 11: a schematic longitudinal sectional view of a diffuser;
- Fig. 12: a schematic longitudinal sectional view of a duct with a tapered inlet section;
- Fig. 13: a schematic sectional view of a dispensing device and storage device with a multiple powder jet impinging means;
- Fig. 14: a schematic sectional view of another multiple powder jet impinging means;
- Fig. 15: a schematic sectional view of a storage device with a further multiple powder jet impinging means;
- Fig. 16: a schematic partial view of another storage device;
- Fig. 17: a schematic partial sectional view of a further storage device;
- Fig. 18a, 18b: schematic partial sectional views of a still further storage device before and during dispensing powder;
- Fig. 19: a schematic sectional view of a dispensing device according to another embodiment;
- Fig. 20: a schematic view of a storage device with radial ducts;
- Fig. 21: a schematic partial sectional view of another dispensing device and storage device; and
- Fig. 22: a schematic sectional view of a further dispensing device and storage device.

In the Fig., the same reference signs are used for same or similar components, wherein same or similar characteristics, features or advantages are or can be realized or achieved even if a repeated discussion is omitted. Further, the features and aspects of the different embodiments can be combined in any desired manner and/or used for other dispensing devices or methods for dispensing powder as desired.

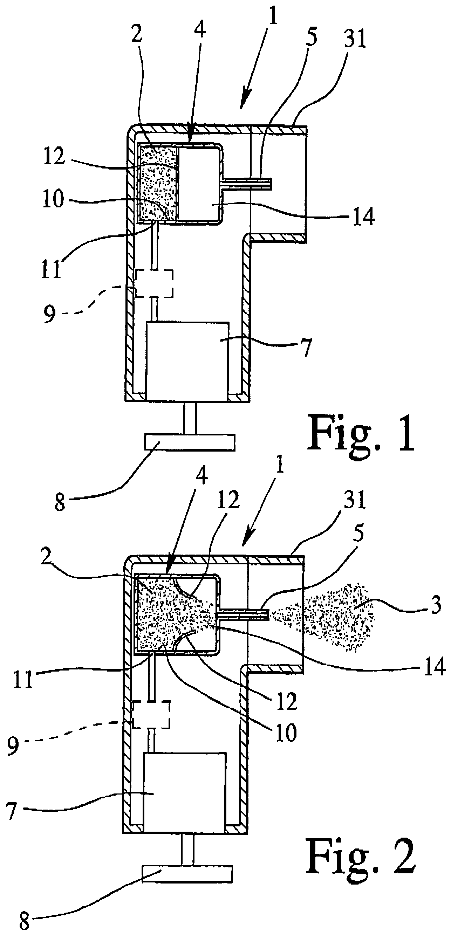

Fig. 1 shows in a schematic cross section - for illustration purposes not in scale - a dispensing device 1. The dispensing device 1 is an active device, in particular gas powered. Preferably, the dispensing device 1 is an inhaler, in particular a dry powder inhaler, for a user or patient (not shown).

The dispensing device 1 is designed to dispense powder 2 which in particular contains or consists of at least one drug. The powder 2 may be a pure drug or a mixture of at least two drugs. In addition, the powder 2 may contain at least one other material, in particular a carrier such as lactose.

Preferably the mean diameter of the powder particles is about 2 to 7 µm, in particular 6 µm or less. This applies in particular if the powder 2 does not contain any carrier such as lactose.

If the powder 2 contains a carrier, such as lactose, and at least one drug, the powder 2 may have a particle size of 20 to 300 µm, in particular about 30 to 60 µm. However, the de-agglomeration, which will be described later in more detail, may result even in this case in a spray 3 with a smaller particle size, e.g. of about 10 µm or less. In particular, the drug may be separated form the carrier during de-agglomeration so that primarily the drug will be inhaled due to its small particle size of about 2 to 6 µm and the larger carrier will be swallowed when using the dispensing device as an inhaler. Alternatively or additionally, breaking or opening of the carrier is possible during de-agglomeration.

The above diameters mentioned above and below may be understood as mass medium aerodynamic diameters and/or may apply to the particle size or a fraction of the particles of the spray 3.

Fig. 2 shows the dispensing device 1 when dispensing the powder 2 as a spray 3 in a very schematic manner similar to Fig. 1. The spray 3 comprises fine (powder) particles, i.e. has fine particle size of preferably 6 µm or less. In particular, the spray 3 has the desired spray plume characteristics as described above.

The dispensing device 1 is adapted to receive or comprises a storage device 4 for storing the powder 2. The storage device 4 may be integrated into the dispensing device 1 or form part of the dispensing device 1. Alternatively, the storage device 4 may be a separate part, in particular a container, cartridge, blister or the like that can be inserted or connected with the dispensing device 1 and optionally replaced.

The dispensing device 1 or the storage device 4 comprises a duct 5 through which the powder 2 is dispensed for de-agglomerating the powder 2 and/or forming the spray 3.

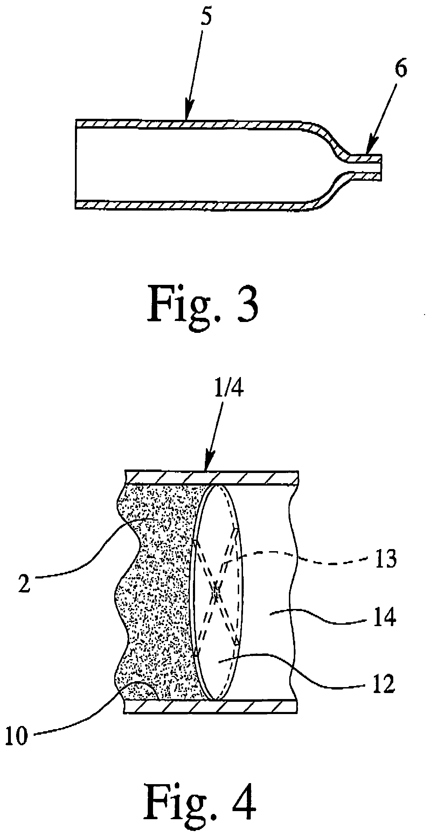

The duct 5 can comprise a nozzle (restriction) 6 preferably at the outlet, as shown in schematic longitudinal cross section according to Fig. 3. Alternatively, the nozzle 6 or any other suitable nozzle arrangement could be used instead or in any other combination with duct 5.

The dispensing device 1 uses pressurized gas to force the powder 2 through the duct 5 / nozzle 6 to de-agglomerate the powder 2 and/or to generate the spray 3 with fine particle size.The dispensing device 1 comprises a means for providing pressurized gas, in the present embodiment an air pump 7 which can preferably be actuated or operated manually as indicated by handle or actuator 8. In particular, the air pump 7 comprises or is formed by a bellows. But, it could be also a piston-cylinder-arrangement. Instead of the air pump 7, the means for providing pressurized gas can be e.g. a capsule, container or the like containing pressurized or liquefied gas for powering the dispensing device 1. i.e. dispensing the powder 2 as desired.

The air pump 7 may provide a gas pressure of less than 300 kPa, in particular about 50 to 200 kPa. This is preferably sufficient for operating the dispensing device 1. If liquefied gas or a container with pressurized gas is used, the gas pressures might range from 100 kPa to about 700 kPa. Then, the pressure may be reduced or throttled to the preferred pressure range before supplying the gas to the storage device 4, in particular its storage chamber 10.

Preferably, all pressure values mentioned in the present description and the claims are gauge pressures, i.e. pressure differences. All pressure values relate to the pressure in a gas storage such as a container with pressurized or liquefied gas or provided by air pump 7 or relate to the pressures acting in the chambers 10, 14 and/or in the duct 5, at least before a bursting element bursts as mentioned below.

The dispensing device 1 optionally comprises a regulation or control means 9 as indicated by dashed lines in Fig. 1 and 2, in particular a valve, a flow restrictor, a capillary tube or the like, for regulating, throttling and/or controlling the gas slow and/or pressure.
The dispensing device 1 or storage device 4 comprises at least one storage chamber 10 containing a single dose of powder 2 that shall be dispensed in one dispensing operation.

For dispensing, the gas is supplied under pressure to the storage chamber 10 / powder 2 via a gas inlet 11 or the like. The inlet I is connected or connectable to the means for providing pressurized gas, i.e. in particular the air pump 7, or to the regulation or control means 9.

In the present embodiment, the storage chamber 10 is preferably covered or closed by a bursting element 12. In particular, the bursting element 12 seals the powder 2 in the storage chamber 10 against humidity, air contact or the like.

When gas is supplied to the storage chamber 10, the bursting element 12 breaks or bursts if a predetermined pressure difference and/or a predetermined velocity of pressure increase is reached or exceeded (generation of a pressure pulse). Preferably, the bursting element 12 is designed to burst at a pressure difference of less than 300 kPa, more preferably about 50 to 200 kPa, and/or at a pressure pulse with a velocity of pressure increase of more than 0.5 MPa/s, in particular more than 1 MPa/s.
The bursting element 12 can be made e.g. of plastic or metal. It can be a thin foil, in particular of aluminum, and/or any suitable membrane.

The bursting element 12 covers a cross sectional area of the storage chamber 10 with a mean diameter of at least 5 mm, preferably 7 mm or more.

Preferably, the bursting element 12 is pre-scratched or pre-scored or comprises at least one weakened portion to facilitate bursting, as indicated by reference sign 13 in Fig. 4 representing an enlarged perspective view of the storage chamber 10 with the bursting element 12. In particular, the bursting element 12 is arranged between the respective storage chamber 10 and an associated mixing chamber 14 as shown in Fig. 4.

When the bursting element 12 bursts or breaks, the storage chamber 10 is opened suddenly and the respective dose of powder 2 is dispensed, namely mixed with the gas, forced through the duct 5 and discharged as spray 3 as shown in Fig. 2.

The gas generates a respective flow in the storage chamber 10 to force all powder 2 through the outlet, i.e. duct 5. The mixing chamber 14 may form an opening space for the optional bursting element 12, so that the bursting element 12 can open sufficiently when it bursts.

The bursting element 12 can be understood as a pressure sensitive element that opens or allows gas flow through the storage chamber 10 for dispensing the respective powder 2 only if a certain gas pressure (peak pressure) is reached or exceeded. However, once the bursting pressure is reached and the bursting element 12 bursts or the pressure sensitive element opens, the gas expands and the pressure will significantly and/or suddenly drop so that the powder 2 may be mixed with the gas during a pressure pulse in a very effective manner, in particular swirls or eddies support the mixing of gas and powder 2, but the powder 2 will be discharged - in particular forced through the duct 5 - with a lower, comparatively low gas pressure (preferably less than 300 kPa, in particular about 50 to 200 kPa). This low gas pressure which is significantly lower than the gas pressures in the prior dispensing devices enables a respectively low discharge velocity and, therefore, a low spray 3 with low propagation velocity.

It has to be noted that the effect of the bursting element 12 can also be realized or supported or even increased by any other suitable pressure sensitive element, in particular by a valve or by a respectively dimensioned reservoir for pressurized gas or the like.

The sudden expansion of the gas ensures good mixing of the gas and powder 2 in the chambers 10 and 14 and, then, forces the mixture through the duct 5.

As already mentioned, the bursting element 12 is only optional. In some cases, the bursting element 12 is not required. Instead, a sealing element or the like may be provided which can be opened e.g. just before dispensing the powder 2. Alternatively, the outlet end of the duct 5 and/or nozzle 6 could be closed for sealing the powder 2 in the storage chamber 10. Only for the dispensing operation, the duct 5 / nozzle 6 is opened, e.g. by cutting, piercing or the like, and then the powder 2 can be discharged.

According to another alternative, the duct 5 / nozzle 6 could be closed by a suitable plug or cap which seals the powder 2 in the storage chamber 10 during non-use. This plug or cap could be removed just before the dispensing operation or opened by the gas pressure during the dispensing operation and have a similar function as the bursting element 12. In order to prevent inhalation of the plug, a respective filter could be provided or the plug could be connected in any suitable manner with another part so that the plug can not separate totally.

Preferably the storage device 4 forms the mixing chamber 14 for mixing the gas with the powder 2. The chambers 10 and 14 are preferably designed such that the gas can generate swirls or eddies for better mixing the powder 2 with the gas. Preferably, the chambers 10 and 14 are substantially circular in cross section, in particular cylindrical. However, other shapes are also possible.

Further, the storage device 4, in particular the chambers 10 and 14 are formed with no sharp edges, corners or the like, but have a smooth contour so that the gas can sweep all chamber surfaces to prevent powder 2 accumulating on said surfaces and to ensure or allow complete discharge of the powder 2. In particular, the gas inlet 11 is located opposite to the powder outlet, i.e. duct 5 and/or nozzle 6, with regard to the axial or outlet direction.

The storage device 4 may comprise only one storage chamber 10 for a single dose, in this case the storage device 4 is for single dose only, or may comprise multiple storage cavities 10 and, thus, contain multiple doses of powder 2, which can be dispensed subsequently.

The gas supply provided by the dispensing device 1, in particular air pump 7, can be connected in any suitable manner to the respective storage device 4 or storage chamber 10, in particular to the respective gas inlet 11, preferably only temporarily when required for a dispensing operation. For example, a piercing element, connecting element or the like can be fluidically connected with gas inlet 11 / the respective storage chamber 10, in particular by pushing it through a respective sealing element, diaphragm, membrane, wall portion or the like to open or enable gas supply to the respective storage chamber 10.

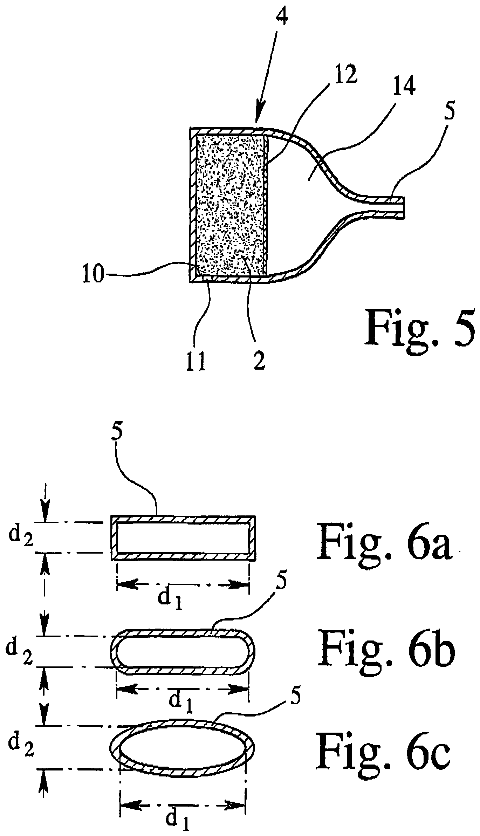

Fig. 5 shows in a very schematic sketch another embodiment of the storage device 4 or storage chamber 10, wherein the side walls, in particular the ones of the mixing chamber 14, or the cross section is tapered towards the duct 5 in order to avoid any sharp edges, corners or the like where the powder 2 may remain despite the gas flow.

According to one aspect of the invention, the duct 5 has a flat (inner) cross section. Fig. 6a to 6c show potential cross sections of the duct 5. Fig. 6a shows a substantially rectangular cross section. Fig. 6b shows a flat cross section with two opposite straight sides connected by two curved portions. Fig. 6c shows an oval or elliptical cross section.

In the present invention, a cross section is considered to be flat when the ratio of the largest side d1 to the smallest side d2 of the cross section is at least 2.0. Preferably, the ratio is between 3 to 50 and in particular about 5 to 70. It is pointed out that the cross sections shown in Fig. 6 are not in scale.

The largest side d1 is preferably between 0.5 to 5 mm, in particular 1 to 3 mm. Most preferably, the ratio of the largest side d1 to the (desired) fine particle size (mass mean diameter of the powder particles or drug particles of the spray 3) is less than 500, preferably less than 300, in particular about 30 to 300.

The smallest side d2 is preferably between 0.05 to 0.5 mm, in particular about 0.07 to 0.25 mm. Most preferably, the ratio of the smallest side d2 to the mass mean (desired) fine particle size (mass mean diameter of the powder particles / drug particles of the spray 3) is less than 50, preferably less than 30, in particular about 3 to 20.

The length of the duct 5 means the length with the flat cross section. Thus, the duct 5 can have a larger length, i.e. further portions with another cross sectional shape and/or with a larger cross sectional area so that the influence of these other portions is low on the mixture of gas and powder 2 in comparison to the portion of the duct 5 with the flat cross section. However, the cross section area and/or the shape of the flat cross section may vary over the length of the duct 5 (the portion with the flat cross section). Thus, it is possible that the cross sectional area of the duct 5 tapers from the inlet to the outlet or vice versa.

Most preferably, the duct 5 comprises at least one portion of flat cross section with constant cross section area, i.e. constant diameter and/or shape.

The length of the duct 5 - i.e. the portion with flat cross section - may be in the range of 3 mm to 80 mm, in particular 5 to 15 mm. Preferably, the duct length is adapted to the mean hydraulic diameter of the duct 5 such that the ratio of the length of the duct 5 to the mean hydraulic diameter is at least 5, in particular about 10, preferably 20 to 60, or more, wherein the hydraulic diameter is defined as the ratio of four cross sectional areas over the duct perimeter.

The diameter of the preferably circular or cylindrical or conical chambers 10 and 14 depend on the volume of mass of the respective dose of powder 2. A single dose may have e.g. 1 to 2 mg (pure drug without carrier) or 2 to 10 mg (blend of drug with carrier, in particular lactose). In the first case, the range of the diameter is preferably 1.5 to 2.5 mm. In the second case, the range of the diameter is preferably between 2 and 5 mm. Preferably, the cross section of the duct 5 varies in a similar manner. For example, the smallest side d2 is about 0.07 to 0.1 mm in the first case and about 0.15 to 0.25 mm in the second case. The larger (inner) side d1 does not depend so strongly on the powder or particle size. Preferably, it is in the range of about 1 to 2 mm in the first case and 1 to 3 mm in the second case.

The mean hydraulic diameter of the duct 5 is preferably less than 1 mm, in particular 0.1 mm to 0.6 mm.

Preferably, the duct 5 is moulded and/or formed by a flat groove with a cover.

The dispensing device 1 or storage device 4 may comprise multiple ducts 5 for dispensing simultaneously one dose of powder 2, in particular for increasing the total mass flow or output of dispensed powder 2 so that a desired dose can be discharged or dispensed in a sufficiently short time as desired and/or required.

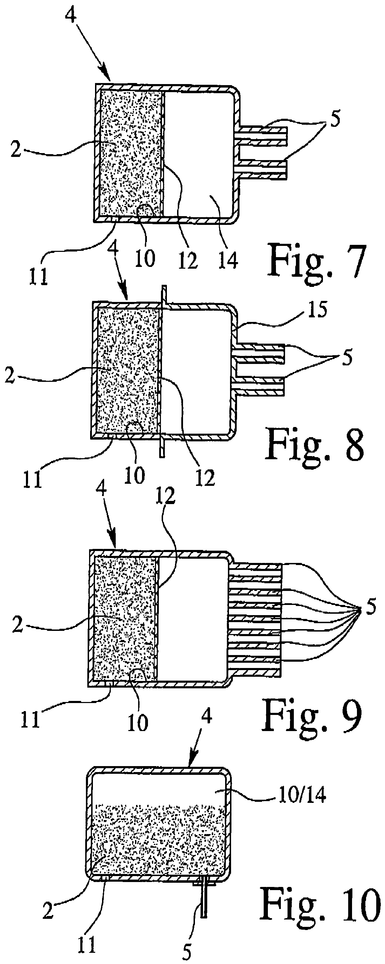

Fig. 7 shows another embodiment of the storage device 4 in a schematic representation similar to Fig. 5. Here, two ducts 5 are associated or connected to one storage chamber 10 / mixing chamber 14.

Fig. 8 shows in a similar representation a further embodiment, wherein the mixing chamber 14 and the ducts 5 are formed by a part separate from the storage device 4 and the storage chamber 10. This separate part can form a cover 15 or the like and is placed in contact with the storage device 4 or over the bursting element 12 before gas is supplied and the powder 2 is dispensed. The separate part may comprise only one duct 5 or multiple ducts 5, in particular two ducts 5 as shown. Multiple ducts 5 have the advantage that even if one duct 5 is blocked, the dispensing device 1 still works, e.g. powder 2 can be still dispensed.

When the storage device 4 comprises multiple storage cavities 10 with respective doses of powder 2, the separate part covering the outlet side of the storage device 4 can be moved from one storage chamber 10 or 14 to the next one for subsequent discharge of the powder 2 from one storage chamber 10 after the other.

Fig. 9 shows another embodiment similar to Fig. 7, but with a multiplicity of ducts 5 which are preferably arranged one directly at the side of the other so that a filter is formed wherein the filter pores are formed by the ducts 5.

Fig. 10 shows a different embodiment of the storage device 4 without bursting element 12. Instead, the storage chamber 10 is closed by a sealing element or the like that can be opened, e.g. pierced, by the duct 5 (as shown in Fig. 10) or in any other suitable manner and connected with the duct 5 and/or nozzle 6. In this embodiment, a separate mixing chamber 14 is not provided. Instead, the storage chamber 10 has a volume that is not completely filled by the powder 2 so that the gas and the powder 2 can be mixed directly within the storage chamber 10, i.e. the storage chamber 10 also forms the mixing chamber.

Another aspect of this embodiment is that the direction of gas supply and the outlet of the mixing chamber 14 do not have to be transversal as it is the case in the previous embodiments. Instead, the gas inlet direction and the outlet direction from the storage chamber 10 may be in parallel planes, but preferably offset to each other and/or parallel to each other.

Fig. 10 shows the dispensing device 1 or storage device 4 with already inserted or connected duct 5. However, in Fig. 5 and 7 to 10 the air supply is not yet connected to the storage chamber 10 or its inlet 11.

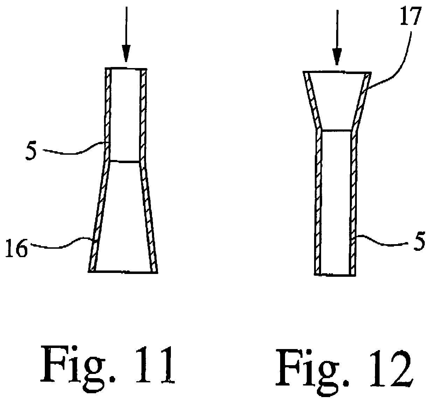

Fig. 11 shows a longitudinal sectional view of another embodiment of the duct 5. Here, the dispensing device 1 or in particular the duct 5 comprises a means for slowing down the outlet velocity and, thus, the propagation velocity of the spray 3. In this embodiment, the means for slowing down the velocity is a diffuser 16 located at or connected to the exit of the duct 5. The diffuser 16 has an appropriate angle to decrease the outlet velocity.

Additionally or alternatively, the duct 5 can also comprise a tapered inlet section 17 as shown in Fig. 12 in a longitudinal section view similar to Fig. 11. The tapered cross section 17 can have any suitable inner contour and may be curved to avoid any sharp edges at the transition to the taper section 17 or from the tapered section 17 to the duct 5. This tapered inlet section 17 may be used for all embodiments with a duct 5 or multiple ducts 5. This applies also for the embodiment according to Fig. 11. i.e. the means for slowing down the outlet velocity.

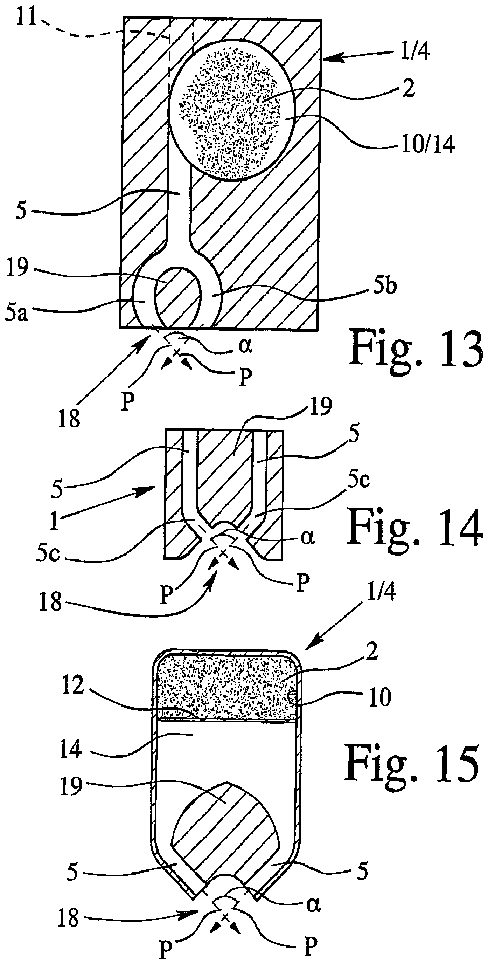

Fig. 13 shows in a schematic sectional view another duct arrangement with another means for slowing down the velocity which forms a multiple powder jet spray impinging means 18. The means 18 forms multiple - at least two - powder spray jets P which impinge, i.e. hit each other as indicated in Fig. 13. In this embodiment, the duct 5 divides into two sections 5a and 5b that are designed such that the openings or outlets are inclined to each other so that the powder jets P ejecting from the portions 5a and 5b are inclined to each other and impinge. For example, a flow divider 19 or any guiding means can be located in the flow path to form the at least two sections 5a and 5b of the duct 5 as shown in Fig. 13.

The impinging angle α between the powder jets P is between 30° to 180°, preferably at least 90°, in particular about 90° to 150°. The impinging of the powder jets P results in a decrease of the velocity of the spray 3 and/or in a de-agglomeration of the powder 2 and/or in separation of drug particles from a carrier and/or in better focusing of the spray 3. These effects depend on the impinging angle α. A larger impinging angle α results in better effects. In contrast to liquid jets, an impinging angle α of 90° and more is possible and preferred. These angles also apply for the following embodiments.

The duct 5 is at least tangentially connected to the storage chamber 10 / mixing chamber 14 in the embodiment shown in Fig. 13. The duct 5 is connected to the mixing chamber 14 at one axial end of the cylindrical chamber 14, and the gas inlet 11 is connected to the other axial end of the chamber 10. The gas inlet 11 is connected also tangentially to the storage chamber 10, such that swirls are generated when entering the gas with a swirl direction supporting discharge of the mixture of gas and powder 2 through the duct 5 which connects tangentially to the rotational direction of the swirl.

Fig. 14 shows in a schematic sectional view another embodiment of the powder jet impinging means 18. Here, two or more ducts 5 comprise inclined or outlet sections 5c which are inclined to each other so that the powder jets P ejected from outlet sections 5c impinge with each other.

Fig. 15 shows in a schematic sectional view a further embodiment of the powder spray jet impinging means 18. Here, the ducts 5 are generally inclined to each other to achieve the designed impingement of the powder jets P. As shown, it is possible that the inlets of the inclined ducts 5 open at the side wall of associated mixing chamber 14 or storage chamber 10. The optional flow divider 19 may be located within the mixing chamber 14 or storage chamber 10 to ensure a desired flow and mixture of the gas with the powder 2.

The embodiments according to Fig. 13 to 15 are also suitable for impinging more than two powder jets P. For example, it is possible to have similar arrangements in the cross sectional planes perpendicular to the drawing plane resulting in four outlet directions and powder jets P arranged on the surface of a conus. However, multiple other arrangements with similar effects are possible.

It has to be added that the cross sections of the duct sections 5a to 5c are preferably not flat but can have any suitable cross sectional shape.

According to another (not shown) embodiment, the duct 5 can also be used as a reservoir (storage chamber 10) for the powder 2. In this case, the separate storage chamber 10 and mixing chamber 14 are not required. In this case, the duct 5 is designed to enable sufficient mixing of the gas with the powder 2 and sufficient de-agglomeration of the powder 2.

Preferably, the gas inlet 11 or the gas supply comprises a smaller cross sectional area than the duct 5 or nozzle 6 so that the gas flow is determined by the inlet and not by the outlet side, i.e. not by the duct 5 or nozzle 6, during dispensing. Thus, the pressure reaches a peak value at the beginning of the dispensing operation just before the bursting element 12 or another pressure sensitive element opens. With opening the bursting element 12 or any other pressure sensitive element, the sudden pressure decrease results in that the gas and powder 2 mix very well and quickly in the storage chamber 10 and mixing chamber 14. Then, the mixture of gas and powder 2 is forced by the already lower gas pressure through the duct 5 and/or any other suitable outlet such as nozzle 6, wherein the gas flow is controlled during this phase at least mainly by the cross section of the gas inlet 11 or any other restriction stream up of gas inlet 11. Due to the comparatively low gas pressure for discharging the powder 2 through duct 5 and/or nozzle 6 a low discharge velocity and, thus, a low propagation velocity of the spray 3 can be achieved. A good de-agglomeration of the powder 2 can be achieved by the flat cross section of the duct 5 even with a comparatively low discharge velocity in the duct 5. In addition, the means for slowing down the propagation velocity of the spray 3, in particular the diffuser 16 or the powder jet impinging means 18, can be used to further decrease the propagation velocity of the spray 3.

Preferably, the spray 3 has a mean velocity (taken 10 cm from the outlet / mouthpiece) of less than 2 m/s, in particular less than 1 m/s. Preferably, the mean duration of the spray 3 is at least 0.2 or 0.3 s, in particular about 0.5 to 2 s.

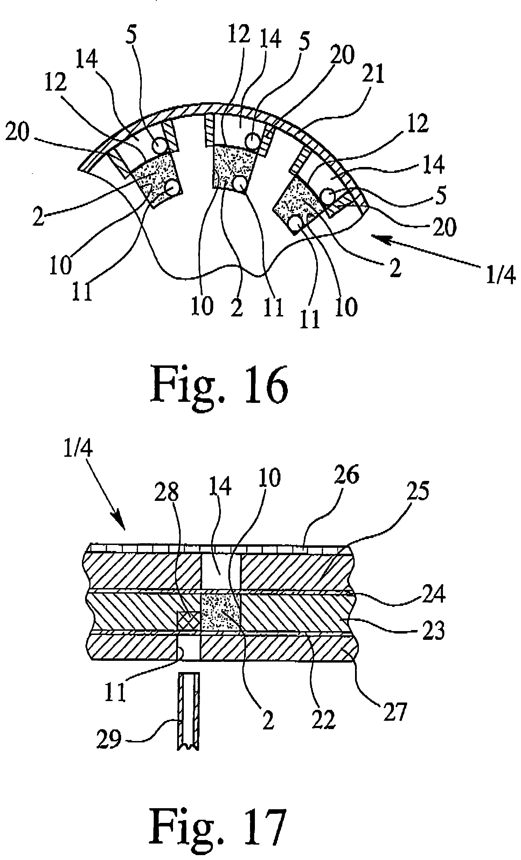

Fig. 16 shows another embodiment of the dispensing device 1 or storage device 4 with multiple storage cavities 10. In particular, the storage device 4 is a circular disc, wherein the storage cavities 10 are positioned along the perimeter of the disc spaced to each other, preferably with a radial orientation so that the associated mixing chambers 14 are located radially outwardly and/or adjacent to the storage cavities 10 and separated by respective bursting elements 12.

The disc may be of molded plastic or made in any other suitable manner or of any other suitable material. The storage cavities 10 and/or the mixing chambers 14 can have a cylindrical or conical form. The bursting elements 12 are preferably disc-like and secured for example by conical holders 20 or the like in place covering the respective storage chamber 10 preferably radially. The holders 20 may be secured e.g. by an outerring 21 or the like.

Fig. 16 shows the disc without an upside cover or with a transparent cover that covers and closes the storage cavities 10 and the mixing chambers 14 from above in the drawing. However, the gas inlets 11 and the duct 5 or other suitable outlet ports are formed in the cover and/or the disc or inserted just for the dispensing operation as depicted in Fig. 16. Alternatively, respective inlet ports and/or ports are formed in the disc. For dispensing a dose of powder 2, the gas inlet 11 or a respective connection element and the duct 5 or a respective connection element are pushed through the sealing cover in the respective ports. Then the gas supply is open or the air pump 7 is actuated, the bursting element 12 bursts and the gas pressure forces the gas powder mixture through the connected duct 5 and/or nozzle 6. Afterwards or just before the next dispensing operation, the disc is rotated to the next position.

Fig. 17 shows a partial axial section of a similar dispensing device 1 or storage device 4 with multiple powder reservoirs (storage cavities 10). Preferably, it's a circular or cylindrical arrangement with some layers or discs placed axially one above the other. The powder 2 is completely from atmospheric humidity (this applies to each embodiment) and, thus kept dry.

In the shown embodiment, a first sealing layer 22, preferably made of foil, in particular a thin coated aluminum foil, acts as a humidity barrier and covers the powder 2 in the respective storage cavities 10 on one axial side. The storage cavities 10 may be formed in a first disc 23, e.g. made of plastic. The first sealing layer 22 is preferably bonded to the first disc 23. At the other side, a second sealing layer 24 is bonded to the first disc 23 and covers the storage cavities 10 from the other side and, thus, forms the bursting elements 12. The second layer 24 is preferably also a foil, in particular a thin coated aluminum foil or the like, and acts as a second humidity barrier for isolating the powder 2 in the storage cavities 10.

The second sealing layer 24, in turn, may be covered by second disc 25 with recesses forming the respective mixing chambers 14 located axially above the associated storage cavities 10 separated by the second sealing layer 24.

A filter layer 26 or any other suitable cover may be bonded on the second disc for covering the mixing chambers 24. This layer 26 or cover may directly form the ducts 5, the nozzle 6 or any other suitable outlet means, such as a mesh, or may be a support and/or seal for an outlet element, such as the duct 5, that can be inserted into the respective mixing chamber 14 through layer 26 for the respective dispensing operation.

A third disc 27 may be bonded to the first sealing layer 22 on the opposite to first disc 23. This third disc 27 can be made e.g. or plastic and comprise recesses or the like forming the gas inlets 11. These gas inlets 11 are preferably offset with respect to the storage cavities 10 so that transversal or tangential gas supply is possible instead of an axial gas supply.

Soft sealing elements 28 are preferably arranged in the first disc 23 opposite to the gas inlet 11. For the dispensing operation, a gas supply element 29 or any other suitable element is pushed into one gas inlet 11 and through the first sealing layer 22 until the corresponding soft sealing element 28 is sufficiently deformed to allow ingress of gas into the associated (adjacent) storage chamber 10. When the gas supply is opened, e.g. regulation or control means 9, or the air pump 7 is actuated and the pressure reaches a peak level, the respective bursting element 12 formed by the second layer 24 and the gas and powder mixture is forced through filter layer 26 or any other suitable output element, such as a duct 5 or nozzle 6.

It has to be pointed out that the third disc 27 is optional. For example, the gas inlets 11 could be formed in the first disc 23, in particular between the first sealing layer 22 and the soft sealing element 28, or omitted.

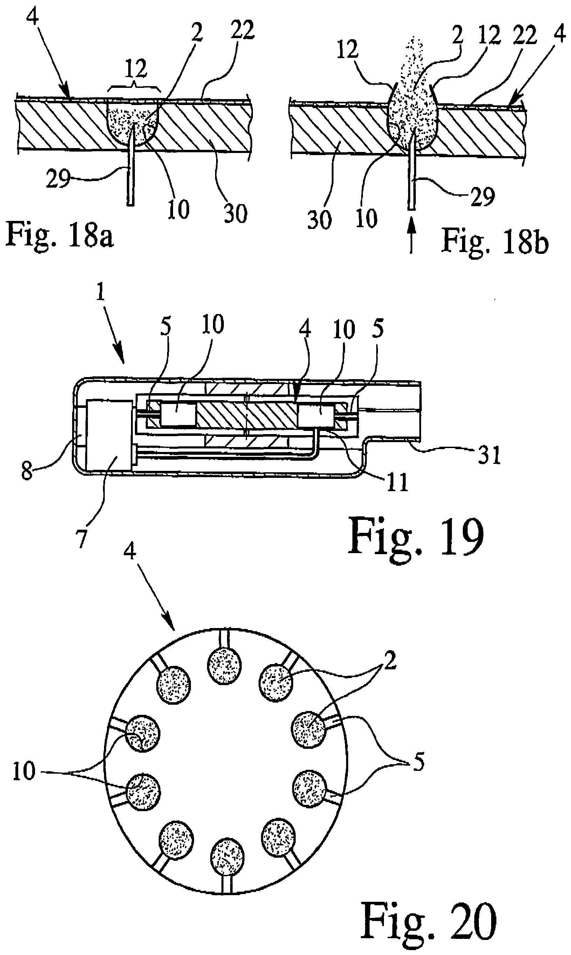

Further, it is possible to puncture or open a wall of the storage chamber 10 directly as shown in another embodiment according to Fig. 18a and 18b. The dispensing device 1 or storage device 4 comprises a base 30 with at least one storage chamber 10 or multiple storage chambers 10. In the latter case, the storage chambers 10 are preferably arranged in a row, wherein the base 30 may form a stripe, or along a circle, wherein the holder 30 may form a disc. A first sealing layer 22 covers the storage chamber(s) 10 and acts as a humidity barrier so that the powder 2 is isolated from the atmosphere and kept dry.

For the dispensing operation, the gas supply element 29 is pushed - for example axially - through the base 30 or through a side wall or through the sealing layer 22 into the respective storage chamber 10 as shown in Fig. 18a, When gas is supplied and the gas pressure exceeds the bursting pressure, the layer 22 forming a bursting element 12 bursts and the powder 2 is discharged as shown in Fig. 18b. The powder 2 could be discharged into a cover 15 with at least one duct 5 as shown in Fig. 8, if that cover 15 is placed on the layer 22 above the respective storage chamber 10 before the gas is supplied.

Fig. 19 shows another embodiment of the dispensing device 1 in a very schematic sectional view. In this embodiment, the storage device 4 is a preferably disc-like cartridge, container, blister or the like with multiple storage chambers. The storage device 4 can be rotated or indexed stepwise so that the powder 2 can be dispensed from the storage chambers 10 one after the other. In this embodiment, the gas may be supplied axially, and the mixture of gas and powder 2 may be dispensed radially, in particular into a mouthpiece 31 for a user or patient (not shown). Preferably, the powder 2 is dispensed through at least one duct 5 and/or a nozzle 6 which is located within the mouthpiece 31 and, in particular, set back with regard to the opening of the mouthpiece 31. This applies preferably also to the dispensing device 1 shown in Fig. 1 and 2.

Fig. 20 shows a further embodiment of the storage device 4 with multiple storage chambers 10 in a circular or disc-like arrangement which could be used in the dispensing device 1 according to Fig. 19.

The storage device 4 comprises ducts 5 which may be oriented radially as shown in Fig. 20. This arrangement has the advantage that each dose of powder 2 may be dispensed through a separate, i.e. unused duct 5. If desired, a bursting element 12 may be arranged between each storage chamber 10 and the respective duct 5 as shown in Fig. 20. Further, a mixing chamber 14 may be associated to each storage chamber 10, in particular between the bursting element 12 and the duct 5, if desired. Alternatively, each storage chamber 10 may also form the mixing chamber as already explained with respect to the embodiment according to Fig. 10. It is pointed out, that the features of the other embodiments, such as the powder jet impinging means 18 or the like, can also be realized in the embodiment according to Fig. 20.

Gas can be supplied in particular axially or tangentially into the storage chambers 10, e.g. by inserting a respective gas supply element 29 or the like.

It has to be noted that in particular the dispensing device 1 and/or the storage device 4, can be used for dispensing one drug, a blend of drugs or at least two or three separate drugs. In the latter case, the separate drugs are stored in separate storage chambers 10 and, during the dispensing operation, the drugs are mixed either in a common mixing chamber 14 or in separate mixing chambers 14 or in their respective storage chambers 10 with the gas. Further, the separate drugs can be discharged through a common duct 5 nozzle 6 or through separate ducts 5 nozzles 6. In the latter case, the separate drugs will be mixed after leaving the separate ducts 5 / nozzles 6 or in the mouthpiece 31 or in any other suitable (additional) mixing chamber. It is also possible to mix the separate drugs by impinging powder jets of the separate drugs. For dispensing the separate drugs, it is possible to use a common gas supply or means for pressurizing gas such as the air pump 7 or separate gas supplies / means for providing pressurized gas.

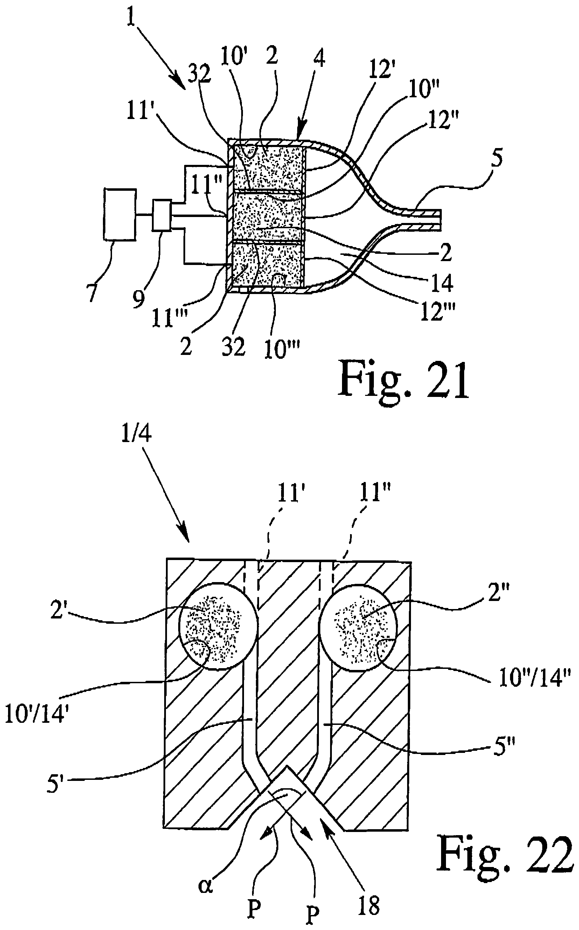

Fig. 21 shows a further embodiment of the dispensing device 1 / storage device 4 with at least two or three separate powders 2, i.e. separate drugs, which are stored in separate storage chambers 10', 10" and 10 "', which may be formed by intermediate walls 32 and each storage chamber can be covered by an optional bursting element 12', 12" or 12"'. Pressurized gas can be supplied from a common means for providing pressurized gas, in particular air pump 7 and/or regulation of control means 9, via separate gas inlets 11', 11" and 11"'. Alternatively, separate gas supplies can be provided.
When the gas pressure reaches the peak or burst pressure in the storage chambers 10', 10" and 10'", the respective bursting elements 12', 12" and 12'" burst and the separate drugs / powders 2', 2" and 2"' can mix in the common mixing chamber 14 and the mixture is dispensed together through at least one common duct 5, nozzle 6 or the like as spray 3 (not shown).

It has to be noted that the bursting elements 12', 12" and 12"' can be formed from one common layer of material, in particular foil or the like.
Alternatively, the central storage chamber 10" could be encompressed by at least one, preferably annular storage chamber. In this case, wall 32 can be cylindrical and the reference signs 10' and 10"' denote the same annular storage chamber resulting in only two different drugs / powders 2 in the two separate storage chambers (central chamber and annular chamber) in the embodiment of fig. 21. In this case, reference signs 12' and 12"' relate to the same annular bursting element covering the annular chamber.

Fig. 22 shows in a schematic sectional view another embodiment of the dispending device 1 and storage device 4. Two different drugs / powders 2' and 2" are contained in separate storage mixing chambers 10'/14' and 10"/14", respectively. Gas can be supplied via separate gas inlets 11' and 11" to the chambers 11'/14' and 10"/14", respectively. The powders 2' and 2" can be dispensed and de-agglomerated by means of separate ducts 5' and 5" or (not shown) separate nozzles or the like. The powder jets P of the separate or different drugs / powders 2' and 2" ejecting from the separate ducts 5' and 5" are preferably impinged for mixing the separate drugs / powders 2' and 2" just when forming the spray 3 (not shown). The powder jet impinging means 18 mixes the separate drugs / powders 2' and 2", but can also serve to slow down the propagation velocity of the spray 3 and/or to support de-agglomeration of the powders 2' and 2" or of separating the respective drugs from carriers.

In the following, two examples are described which show the effect of the present invention.

Example 1: A blend of 90.0 % by weight of lactose 200, of 9.7 % by weight of fine lactose, and of 0.3 % by weight of Tiotropium was used. The mean particle diameter of lactose 200 was about 45 µm, of fine lactose about 4 µm and of Tiotropium about 4 µm. About 5.5 mg of the blend was positioned as powder 2 in the storage and mixing chamber 10 / 14 which had a substantially cylindrical shape with a diameter of 3 mm and an axial length of 3 mm. 5 ml of compressed air was supplied via the gas inlet having an inlet orifice of 0.5 mm into the chamber 10/14 with a gauge pressure of about 100 kPa. The powder 2 was dispensed via duct 5 of substantially rectangular cross section having a smallest side of about 0.18 mm and a largest side of about 1.5 mm. The duct 5 divided into two duct sections 5a and 5b (in particular as shown in Fig. 13), wherein each section had a substantially rectangular cross section with a smallest side of about 0.18 mm and the largest side of about 0.75 mm. The total length of the duct 5 including the sections 5a, 5b was about 8 mm. The result was that 100 % of the metered mass, i.e. all powder 2 in chamber 10 / 14, was dispensed. Approximately 50% of both diameter mean and mass mean fine fraction was measured on a Anderson Cascade Impactor at both 30 and 60 l/min.

Example 2: About 1.5 mg of Fenoterol with a mean particle diameter of 4 µm was positioned as powder 2 in the storage and mixing chamber 10 / 14 which had a substantially cylindrical shape with a diameter of 2 mm and an axial length of 2 mm. 5 ml of compressed air was supplied via the gas inlet having an inlet orifice of 0.5 mm into the chamber 10/14 with a gauge pressure of about 150 kPa. The powder 2 was dispensed via a duct 5 of substantially rectangular cross section having a smallest side of 0.075 mm and a largest side of 1.5 mm. The duct 5 divided into two duct sections 5a and 5b (in particular as shown in fig. 13), wherein each section had a substantially rectangular cross section with a smallest side of about 0.075 mm and the largest side of about 0.75 mm. The total length of the channel including the sections 5a, 5b was about 8 mm. The result was that 100 % of the metered mass, i.e. all powder 2 in chamber 10 / 14, was dispensed. Approximately 45% of both diameter mean and mass mean fine fraction was measured on a Anderson Cascade Impactor at both 30 and 60 l/min.

Further aspects are:
1. Dispensing device (1) for dispensing powder (2), in particular containing or consisting of a drug, as a spray (3) including fine powder particles, the dispensing device (1) comprising a duct (5) through which the powder (2) is dispensable by gas pressure for de-agglomerating the powder (2),
   characterized in
   that the duct (5) has a flat cross section, the ratio of the largest side (d1) to the smallest side (d2) of the flat cross section being at least 2.0.
2. Dispensing device according to aspect 1, characterized in that the ratio is between 3 to 50, preferably about 5 to 30.
3. Dispensing device according to aspect 2, characterized in that the largest side (d1) is between 0.5 to 5 mm, preferably about 1 to 3 mm.
4. Dispensing device according to any one of the preceding aspects, characterized in that the ratio of the largest side (d1) to is the size of the fine powder particles is less than 500, preferably less than 300, in particular about 30 to 300.
5. Dispensing device according to any one of the preceding aspects, characterized in that the smallest side (d2) is between 0.05 to 0.5 mm, preferably about 0.07 to 0.25 mm.
6. Dispensing device according to any one of the preceding aspects, characterized in that the ratio of the smallest side (d2) to the size of the fine powder particles is less than 50, preferably less than 30, in particular about 3 to 20.
7. Dispensing device according to any one of the preceding aspects, characterized in that the flat cross section is substantially oval or rectangular.
8. Dispensing device according to any one of the preceding aspects, characterized in that the dispensing device (1) comprises means for providing pressurized gas, in particular air, for forcing the powder (2) through the duct (5) and/or dispensing the powder (2).
9. Dispensing device according to any one of the preceding aspects, characterized in that the dispensing device (1) is adapted to receive or comprises a replaceable storage device (4) with a single dose of powder (2) or with multiple separate doses of powder (7), so that the doses are dispensable subsequently.
10. Dispensing device (1) for dispensing powder (2), in particular containing or consisting of a drug, as a spray (3) including fine powder particles, preferably according to any one of the preceding aspects,
   wherein the dispensing device (1) is adapted to receive or comprises a storage device (4) with at least one storage chamber (10) containing one dose of the powder (2), and
   wherein the dispensing device (1) comprises a means for providing pressurized gas, in particular air, for dispensing the powder (2) preferably through a duct (5) or nozzle (6),
   characterized in
   that the storage chamber (10) is closed by a bursting element (12) or another pressure sensitive element designed such that the pressurized gas can burst the bursting element (12) or open the pressure sensitive element for discharging powder (2) from the associated storage chamber (10) to dispense the powder (2), and/or
   that a first cross section of a gas supply or inlet (11) to the storage chamber (10) is smaller than a second cross section of an outlet for the powder (2) to substantially control or restrict the gas flow during dispensing of the powder (2) by the first cross section.
11. Dispensing device according to aspect 10, characterized in that the bursting element (12) is made of plastic or metal and/or of foil.
12. Dispensing device according to aspect 10 or 11, characterized in that the bursting element (12) has a disc-like or oval or polygonal shape.
13. Dispensing device according to any one of aspects 10 to 12, characterized in that the bursting element (12) covers a cross sectional area of the storage chamber (10) with a mean diameter of at least 5 mm, preferably 7 mm or more.
14. Dispensing device according to any one of aspects 10 to 13, characterized in that the bursting element (12) is pre-scratched or pre-scored or comprises at least one weakened portion (13) to facilitate bursting.
15. Dispensing device according to any one of aspects 10 to 14, characterized in that the bursting element (12) is designed to burst at a pressure difference of less than 300 kPa, preferably about 50 to 200 kPa.
16. Dispensing device according to any one of aspects 10 to 15, characterized in that the bursting element (12) is designed to burst at a pressure pulse, in particular with a velocity of pressure increase of more than 0.5 MPa/s, preferably more than 1 MPa/s.
17. Dispensing device according to any one of aspects 9 to 16, characterized in that the storage device (4) comprises multiple separate storage chambers (10) each closed by a bursting element (12) and each containing a dose of powder (2).
18. Dispensing device according to aspect 17, characterized in that the bursting elements (12) are interconnected or formed by a common layer (24).
19. Dispensing device according to any one of aspects 9 to 18, characterized in that the storage device (4) is constructed such that each dose of powder (2) is dispensed through a separate duct (5) or nozzle (6).
20. Dispensing device according to any one of aspects 9 to 19, characterized in that the storage device (4) is a cartridge, blister, capsule or container.
21. Dispensing device according to any one of aspects 9 to 20, characterized in that the storage device (4) is designed according to any one of aspects 34 to 46.
22. Dispensing device according to any one of the proceeding aspects, characterized in that the dispensing device (1) comprises an air pump (7) as means for providing pressurized gas, wherein the air pump (7) is preferably manually operated.
23. Dispensing device according to any one of the preceding aspects, characterized in that the gas gauge pressure for dispensing the powder (2) is less than 300 kPa, preferably about 50 to 200 kPa.
24. Dispensing device according to any one of the preceding aspects, characterized in that the ratio of the length of the duct (5) to the mean hydraulic diameter of the duct (5) is at least 5, preferably about 10 or more.
25. Dispensing device according to any one of the preceding aspects, characterized in that the mean hydraulic diameter of the duct (5) is less than 1 mm, preferably about 0.1 to 0.6 mm.
26. Dispensing device according to any one of the preceding aspects, characterized in that the dispensing device (1) comprises multiple ducts (5) for dispensing simultaneously one dose of powder (2), in particular for increasing the total mass flow of dispensed powder (2).
27. Dispensing device according to any one of the preceding aspects, characterized in that the dispensing device (1) and/or the storage device (4) is constructed such that each dose of powder (2) is dispensed through a separate or unused duct (5).
28. Dispensing device according to any one of the preceding aspects, characterized in that the dispensing device (1) or the storage device (4) is adapted to receive or contains at least two or three powders (2) in separate storage chambers (10), wherein the powders (2) can be mixed preferably only during the dispensing operation.
29. Dispensing device according to any one of the preceding aspects, characterized in that the dispensing device (1) comprises a means for slowing down the propagation velocity of the spray (3), in particular a diffuser (16), preferably at the exit of the duct (5).
30. Dispensing device according to any one of the preceding aspects, characterized in that the dispensing device (1) comprises a powder jet impinging means (18) for impinging at least two powder jets (P) to de-agglomerate the powder (2) and/or to slow down the propagation velocity of the spray (3) and/or to mix separate powders (2).
31. Dispensing device according to aspect 28, characterized in that the impinging angle (α) between the powder jets (P) is between 30° to 180°, preferably at least 90°, in particular about 90°to 150°.
32. Dispensing device according to any one of the preceding aspects, characterized in that the mean size of the powder particles is 2 to 7 µm when the powder (2) is a pure drug or a blend of drugs, or that the mean size of the powder particles is 20 to 300 µm when the powder (2) is a blend of a carrier, such as lactose, with at least one drug.
33. Dispensing device according to any one of the preceding aspects, characterized in that the dispensing device (1) is a dry powder an inhaler.
34. Storage device (4) for powder (2), in particular containing or consisting of a drug, with at least one, preferably multiple separate storage chambers (10) each containing a single dose of the powder (2) to be dispensed by gas pressure,
   **characterized in**
   that the at least one storage chamber (10) is closed by a bursting element (12) designed such that the gas pressure can burst the bursting element (12) for discharging the powder (2) from the associated storage chamber (10) to dispense the powder (2), and/or
   that the storage device (4) comprises ducts (5) and/or nozzles (6) associated to the storage chambers (10) so that each dose of powder (2) can be dispensed through a separate duct (5) or nozzle (6) by the gas pressure.
35. Storage device according to aspect 34, characterized in that the bursting elements (12) are made of plastic or metal and/or of foil.
36. Storage device according to aspect 34 or 35, characterized in that the bursting elements (12) have a disk-like or oval or polygonal shape.
37. Storage device according to any one of aspects 34 to 36, characterized in that the bursting elements (12) are interconnected or formed by a common layer (24).
38. Storage device according to any one of aspects 34 to 37, characterized in that each bursting element (12) covers a cross sectional area of the associated storage chamber (10) with a mean diameter of at least 5 mm, preferably 7 mm or more.
39. Storage device according to any one of the aspects 34 to 38, characterized in that the bursting elements (12) are pre-scratched or pre-scored or comprise at least one weakened portion (13) to facilitate bursting.
40. Storage device according to any one of aspects 34 to 39, characterized in that the bursting elements (12) are designed to burst at a pressure difference of less than 300 kPa, preferably about 50 to 200 kPa.
41. Storage device according to any one of aspects 34 to 40, characterized in that the bursting elements (12) are designed to burst at a pressure pulse, in particular with a velocity of pressure increase of more than 0.5 MPa/s, preferably more than 1 MPa/s.
42. Storage device according to any one of aspects 34 to 41, characterized in that the ratio of the length of each duct (5) to the mean hydraulic diameter of the duct (5) is at least 5, preferably about 10 or more.
43. Storage device according to any one of aspects 34 to 42, characterized in that the mean hydraulic diameter of each duct (5) is less than 1 mm, preferably about 0.1 to 0.6 mm.
44. Storage device according to any one of aspects 34 to 43, characterized in that the ducts (5) have a flat cross section, preferably wherein the ratio of the largest side (d1) to the small side (d2) of the flat cross section is at least 2.0.
45. Storage device according to any one of aspects 34 to 44, characterized in that the storage device (4) is a cartridge, blister or container.
46. Storage device according to any one of aspects 34 to 45, characterized in that the storage device (4) contains at least two or three powders (2) in separate storage chambers (10), wherein the powders (2) can be mixed preferably only during the dispensing operation.
47. Method for dispensing powder (2), in particular containing or consisting of a duct, as a spray (3) including fine powder particles by means of gas pressure,
   characterized in
   that the powder (2) is forced through a duct (5) by a gauge gas pressure of less than 300 kPa to de-agglomerate the powder (2) and/or to generate the spray (3) and/or
   that at least two powder jets (P) are impinged to mix at least two separate drugs or powders (2).
48. Method according to aspect 47, characterized in that a gauge gas pressure of about 50 to 200 kPa is used to de-agglomerate the powder (2) and to generate the spray (3).
49. Method according to aspect 47 or 48, characterized in that the impinging angle (α) between the powder jets (P) is between 30° to 180°, preferably at least 90°, in particular about 90° to 150°.
50. Method for dispensing powder (2), in particular containing or consisting of a drug, as a spray (3) including fine powder particles, preferably according to any one of aspects 47 to 49,
   characterized in
   that the powder (2) located in a storage chamber (10) is subjected to a gas pressure such that an associated bursting element (12) closing the storage chamber (10) bursts or an associated pressure sensitive element opens, when the gas pressure reaches or exceeds a peak value, for sudden mixing the powder (2) with the gas and subsequent discharging the powder (2) from the storage chamber (10) to dispense the powder (2), and/or
   that the gas pressure reaches or exceeds a peak value before the powder (2) is dispensed and that the gas pressure is lower than the peak value during dispensing the powder (2).
51. Method according to aspect 50, characterized in that a pressure difference of less than 300 kPa, preferably about 50 to 200 kPa, is generated or used to burst the bursting element (12).
52. Method according to aspect 50 or 51, characterized in that a pressure pulse is generated or used to burst the bursting element (12).
53. Method according to aspect 52, characterized in that the pressure pulse has a velocity of pressure increase of more than 0.5 MPa/s, preferably more than 1 MPa/s.
54. Method according to any one of aspects 47 to 53, characterized in that the gas flow is restricted or determined by the cross section of a gas supply or inlet (11) stream up of the powder (2) during dispensing the powder (2).
55. Method according to any one of aspects 47 to 54, characterized in that the ratio of the volume of gas used to dispense the powder (2) at atmospheric pressure to the volume of dispensed powder (2) is about 10000 or less, preferably about 200 to 2000.
56. Method according to any one of aspects 47 to 55, characterized in that air is pressurized and used as gas.
57. Method according to aspect 56, characterized in that air is pressurized by manually operating an air pump (7).
58. Method according to any one of aspects 47 to 57, characterized in that the mean size of the powder (2) is 2 to 7 µm when the powder (2) consists of a pure drug or a blend of drugs, or that the mean size of the powder (2) is 20 to 300 µm when the powder (2) consists of a blend of a carrier, such as lactose, and at least one drug.
59. Method according to any one of aspects 47 to 58, characterized in that at least two or three powders (2) are stored in separate storing chambers (10) and mixed only during the dispensing.

## Claims

1. Dispensing device (1) for dispensing powder (2), in particular containing or consisting of a drug, as a spray (3) including fine powder particles,
the dispensing device (1) comprising,
a means for providing pressurized gas for dispensing the powder (2),
a mouthpiece (31),
a storage device (4) having multiple separate storage chambers (10) each containing a single dose of the powder (2) to be dispensed by gas pressure,
wherein the storage device (4) comprises ducts (5) associated to the storage chambers (10) so that each dose of powder (2) can be dispensed through a separate duct (5) by the gas pressure,
wherein each storage chamber (10) comprises a respective gas inlet (11), wherein the respective duct (5) forms a respective powder outlet,
wherein the duct (5) is connected to the respective storage chamber (10) at one axial end and the gas inlet (11) is connected to the other axial end of the respective storage chamber (10), and
wherein the gas inlet (11) is connected tangentially to the respective storage chamber (10), and wherein the duct (5) is tangentially connected to the respective storage chamber (10),
**characterized in**
**that** the respective duct (5) is located at a mouthpiece entrance with no flow restrictions after the duct (5) for dispensing the powder (2).

2. Dispensing device according to claim 1, **characterized in that** the storage chambers (10) have respectively a volume that is not completely filled by the powder (2) so that the gas and the powder (2) can be mixed directly within the respective storage chamber (10).

3. Dispensing device according to claim 1 or 2, **characterized in that** the ratio of the chamber volume of the respective storage chamber (10) to the powder volume of the respective dose of powder (2) is between 1.2 and 4.

4. Dispensing device according to any one of the preceding claims, **characterized in that** the gas inlet (11) is located opposite to the powder outlet with regard to the axial or outlet direction.

5. Dispensing device according to any one of the preceding claims, **characterized in that** the gas inlet direction and the outlet direction from the storage chamber (10) are in parallel planes, but offset to each other and/or parallel to each other.

6. Dispensing device according to any one of the preceding claims, **characterized in that** the storage chambers (10) are substantially circular in cross section, in particular cylindrical.

7. Dispensing device according to any one of the preceding claims, **characterized in that** the side walls or the cross section of the respective storage chamber (10) are tapered towards the duct (5) in order to avoid any sharp edges.

8. Dispensing device according to any one of the preceding claims, **characterized in that** the outlet end of the duct (5) is closed for sealing the powder (2) in the storage chambers (10), wherein only for the dispensing operation, the respective duct (5) is opened.

9. Dispensing device according to any one of the preceding claims, **characterized in that** the storage chambers (10) are arranged along a circle, wherein the ducts (5) are oriented radially.

10. Dispensing device according to any one of the preceding claims, **characterized in that** the ducts (5) have a flat cross section, preferably wherein the ratio of the largest side (d1) to the small side (d2) of the flat cross section is at least 2.0.

11. Dispensing device according to any one of the preceding claims, **characterized in that** the dispensing device (1) comprises a piercing element or gas supply element (29) fluidically connectable with the respective storage chamber (10), in particular by pushing it through a respective sealing element, membrane or wall portion to open or enable gas supply to the respective storage chamber (10).

12. Dispensing device according to any one of the preceding claims, **characterized in that** the storage device (4) can be rotated or indexed stepwise.

13. Dispensing device according to any one of the preceding claims, **characterized in that** the duct (5) comprises a means for slowing down the outlet velocity and, thus, the propagation velocity of the spray (3), in particular the means forms multiple powder spray jets P which impinge each other.

14. Dispensing device according to any one of the preceding claims, **characterized in that** the duct (5) divides into two sections (5a, 5b) that are designed such that the openings or outlets are inclined to each other.

15. Dispensing device according to any one of the preceding claims, **characterized in that** the gas flow into the storage chamber (10) is restricted or controlled at the inlet side, in particular by a respectively small cross section of the gas inlet (11).

## Patentansprüche

1. Abgabevorrichtung (1) zur Abgabe von Pulver (2), insbesondere enthaltend ein oder bestehend aus einem Arzneimittel, als ein Spray (3), das feine Pulverpartikel umfasst,
wobei die Abgabevorrichtung (1) umfasst,
ein Mittel zum Bereitstellen von Druckgas zur Abgabe des Pulvers (2),
ein Mundstück (31),
eine Vorratseinrichtung (4) mit mehreren separaten Vorratskammern (10), die jeweils eine Einzeldosis des mittels Gasdruck abzugebenden Pulvers (2) enthalten,
wobei die Vorratseinrichtung (4) den Vorratskammern (10) zugeordnete Kanäle (5) umfasst, sodass jede Dosis Pulver (2) mittels Gasdruck durch einen separaten Kanal (5) abgegeben werden kann,
wobei jede Vorratskammer (10) einen jeweiligen Gaseinlass (11) umfasst, wobei der jeweilige Kanal (5) einen jeweiligen Pulverauslass bildet,
wobei der Kanal (5) mit der jeweiligen Vorratskammer (10) an einem axialen Ende verbunden ist und der Gaseinlass (11) mit dem anderen axialen Ende der jeweiligen Vorratskammer (10) verbunden ist, und
wobei der Gaseinlass (11) tangential mit der jeweiligen Vorratskammer (10) verbunden ist, und wobei der Kanal (5) tangential mit der jeweiligen Vorratskammer (10) verbunden ist,
**dadurch gekennzeichnet,**
**dass** sich der jeweilige Kanal (5) an einem Mundstückeingang mit keinen Flussbeschränkungen nach dem Kanal (5) zum Abgeben des Pulvers (2) befindet.

2. Abgabevorrichtung nach Anspruch 1, **dadurch gekennzeichnet, dass** die Vorratskammern (10) jeweils ein Volumen haben, das nicht vollständig mit dem Pulver (2) gefüllt ist, sodass das Gas und das Pulver (2) direkt innerhalb der jeweiligen Vorratskammer (10) gemischt werden können.

3. Abgabevorrichtung nach Anspruch 1 oder 2, **dadurch gekennzeichnet, dass** das Verhältnis des Kammervolumens der jeweiligen Vorratskammer (10) zu dem Pulvervolumen der jeweiligen Dosis des Pulvers (2) zwischen 1,2 und 4 liegt.

4. Abgabevorrichtung nach einem der vorhergehenden Ansprüche, **dadurch gekennzeichnet, dass** sich der Gaseinlass (11) gegenüber dem Pulverauslass in Bezug auf die axiale oder Auslassrichtung befindet.

5. Abgabevorrichtung nach einem der vorhergehenden Ansprüche, **dadurch gekennzeichnet, dass** sich die Gaseinlassrichtung und die Auslassrichtung von der Vorratskammer (10) in parallelen Ebenen, aber versetzt und/oder parallel zueinander, befinden.

6. Abgabevorrichtung nach einem der vorhergehenden Ansprüche, **dadurch gekennzeichnet, dass** die Vorratskammern (10) im Wesentlichen von kreisförmigem Querschnitt, insbesondere zylindrisch, sind.

7. Abgabevorrichtung nach einem der vorhergehenden Ansprüche, **dadurch gekennzeichnet, dass** sich die Seitenwände oder der Querschnitt der jeweiligen Vorratskammer (10) zu dem Kanal (5) hin verjüngen, um jegliche scharfe Kanten zu vermeiden.

8. Abgabevorrichtung nach einem der vorhergehenden Ansprüche, **dadurch gekennzeichnet, dass** das Auslassende des Kanals (5) zum Abdichten des Pulvers (2) in den Vorratskammern (10) geschlossen ist, wobei der jeweilige Kanal (5) nur für den Abgabevorgang geöffnet wird.

9. Abgabevorrichtung nach einem der vorhergehenden Ansprüche, **dadurch gekennzeichnet, dass** die Vorratskammern (10) entlang einem Kreis angeordnet sind, wobei die Kanäle (5) radial ausgerichtet sind.

10. Abgabevorrichtung nach einem der vorhergehenden Ansprüche, **dadurch gekennzeichnet, dass** die Kanäle (5) einen flachen Querschnitt haben, wobei vorzugweise das Verhältnis der längsten Seite (d1) zu der kurzen Seite (d2) des flachen Querschnitts mindestens 2,0 beträgt.

11. Abgabevorrichtung nach einem der vorhergehenden Ansprüche, **dadurch gekennzeichnet, dass** die Abgabevorrichtung (1) ein Stechelement oder Gaszufuhrelement (29) umfasst, das mit der jeweiligen Vorratskammer (10) fluidisch verbindbar ist, insbesondere indem es durch ein jeweiliges Abdichtelement, eine Membran oder einen Wandabschnitt gedrückt wird, um die Gaszufuhr zu der jeweiligen Vorratskammer (10) zu öffnen oder zu ermöglichen.

12. Abgabevorrichtung nach einem der vorhergehenden Ansprüche, **dadurch gekennzeichnet, dass** die Vorratseinrichtung (4) schrittweise gedreht oder weiterbewegt werden kann.

13. Abgabevorrichtung nach einem der vorhergehenden Ansprüche, **dadurch gekennzeichnet, dass** der Kanal (5) ein Mittel zum Verlangsamen der Auslassgeschwindigkeit und somit der Ausbreitungsgeschwindigkeit des Sprays (3) umfasst, insbesondere bildet das Mittel mehrere Pulversprühstrahlen P, die aufeinander prallen.

14. Abgabevorrichtung nach einem der vorhergehenden Ansprüche, **dadurch gekennzeichnet, dass** sich der Kanal (5) in zwei Abschnitte (5a, 5b) aufteilt, die so ausgebildet sind, dass die Öffnungen oder Auslässe einander zugeneigt sind.

15. Abgabevorrichtung nach einem der vorhergehenden Ansprüche, **dadurch gekennzeichnet, dass** der Gasstrom in die Vorratskammer (10) an der Einlassseite begrenzt oder kontrolliert wird, insbesondere durch einen jeweils kleinen Querschnitt des Gaseinlasses (11).

## Revendications

1.Dispositif de distribution (1) pour la distribution de poudre (2), en particulier contenant ou constituée d'un médicament, tel qu'un aérosol (3) contenant des particules de poudre fines,
le dispositif de distribution (1) comprenant,
un moyen de fourniture de gaz sous pression pour distribuer la poudre (2),
un embout buccal (31),
un dispositif de stockage (4) ayant de multiples chambres de stockage séparées (10) contenant chacune une dose unique de la poudre (2) à distribuer par pression gazeuse,
dans lequel le dispositif de stockage (4) comprend des conduits (5) associés aux chambres de stockage (10) de sorte que chaque dose de poudre (2) puisse être distribuée par le biais d'un conduit séparé (5) par la pression gazeuse,
dans lequel chaque chambre de stockage (10) comprend une entrée de gaz respective (11), dans lequel le conduit respectif (5) forme une sortie de poudre respective,
dans lequel le conduit (5) est raccordé à la chambre de stockage respective (10) au niveau d'une extrémité axiale et l'entrée de gaz (11) est raccordée à l'autre extrémité axiale de la chambre de stockage respective (10) et
dans lequel l'entrée de gaz (11) est raccordée de façon tangentielle à la chambre de stockage respective (10) et dans lequel le conduit (5) est raccordé de façon tangentielle à la chambre de stockage respective (10),
**caractérisé en ce**
**que** le conduit respectif (5) est situé au niveau d'une admission d'embout buccal sans restrictions de flux après le conduit (5) pour distribuer la poudre (2).

2. Dispositif de distribution selon la revendication 1, **caractérisé en ce que** les chambres de stockage (10) ont respectivement un volume qui n'est pas complètement rempli par la poudre (2), de sorte que le gaz et la poudre (2) puissent être mélangés directement au sein de la chambre de stockage respective (10).

3. Dispositif de distribution selon la revendication 1 ou 2, **caractérisé en ce que** le rapport du volume de chambre de la chambre de stockage respective (10) au volume de poudre de la dose respective de poudre (2) est entre 1,2 et 4.

4. Dispositif de distribution selon l'une quelconque des revendications précédentes, **caractérisé en ce que** l'entrée de gaz (11) est située à l'opposé de la sortie de poudre par rapport à la direction axiale ou de sortie.

5. Dispositif de distribution selon l'une quelconque des revendications précédentes, **caractérisé en ce que** la direction d'entrée de gaz et la direction de sortie depuis la chambre de stockage (10) sont dans des plans parallèles, mais décalés l'un de l'autre et/ou parallèles l'un à l'autre.

6. Dispositif de distribution selon l'une quelconque des revendications précédentes, **caractérisé en ce que** les chambres de stockage (10) ont une section transversale sensiblement circulaire, en particulier cylindrique.

7. Dispositif de distribution selon l'une quelconque des revendications précédentes, **caractérisé en ce que** les parois latérale ou la section transversale de la chambre de stockage respective (10) sont coniques vers le conduit (5) afin d'éviter tout bord tranchant.

8. Dispositif de distribution selon l'une quelconque des revendications précédentes, **caractérisé en ce que** l'extrémité de sortie du conduit (5) est fermée pour sceller la poudre (2) dans les chambres de stockage (10), dans lequel, uniquement pour l'opération de distribution, le conduit (5) respectif est ouvert.

9. Dispositif de distribution selon l'une quelconque des revendications précédentes, **caractérisé en ce que** les chambres de stockage (10) sont disposées le long d'un cercle, dans lequel les conduits (5) sont orientés de façon radiale.

10. Dispositif de distribution selon l'une quelconque des revendications précédentes, **caractérisé en ce que** les conduits (5) ont une section transversale plate, de préférence dans lequel le rapport du côté le plus grand (d1) au petit côté (d2) de la section transversale plate est d'au moins 2,0.

11. Dispositif de distribution selon l'une quelconque des revendications précédentes, **caractérisé en ce que** le dispositif de distribution (1) comprend un élément de perçage ou un élément de fourniture de gaz (29) pouvant être placé en raccordement fluidique avec la chambre de stockage respective (10), en particulier en le poussant à travers un élément d'étanchéité, une membrane ou une partie de paroi respectif(ve) afin d'ouvrir ou de permettre la fourniture de gaz à la chambre de stockage respective (10).

12. Dispositif de distribution selon l'une quelconque des revendications précédentes, **caractérisé en ce que** le dispositif stockage (4) peut être entraîné en rotation ou indexé par étapes.

13. Dispositif de distribution selon l'une quelconque des revendications précédentes, **caractérisé en ce que** le conduit (5) comprend un moyen pour ralentir la vitesse de sortie et, ainsi, la vitesse de propagation de l'aérosol (3), en particulier le moyen forme des jets d'aérosol en poudre multiples (P) qui se heurtent les uns contre les autres.

14. Dispositif de distribution selon l'une quelconque des revendications précédentes, **caractérisé en ce que** le conduit (5) se divise en deux sections (5a, 5b) qui sont conçues de sorte que les ouvertures ou sorties sont inclinées l'une par rapport à l'autre.

15. Dispositif de distribution selon l'une quelconque des revendications précédentes, **caractérisé en ce que** l'écoulement de gaz dans la chambre de stockage (10) est restreint ou régulé au niveau du côté d'entrée, en particulier par une section transversale respectivement petite de l'entrée de gaz (11).
